# EUROPEAN PATENT SPECIFICATION

(11) **EP 1 621 148 B1**
(45) Date of publication and mention of the grant of the patent: **31.10.2007**
(21) Application number: 05254666.0
(22) Date of filing: 27.07.2005
(51) Int. Cl.: A61B 17/12

(54) **Embolic device deployment system with filament release**
Einführvorrichtung für Emboliespirale mit Bandlösesystem
Dispositif de déploiement d'une spirale embolique avec un filament de détachement

(30) Priority: 30.07.2004 US 592580 P; 03.06.2005 US 145350
(43) Date of publication of application: 01.02.2006
(73) Proprietor: Cordis Neurovascular, Inc., Miami Lakes, Florida 33014 (US)
(72) Inventor: Lulo, Robert, Pembroke Pines FL 33026 (US); Sherman, Darren, Fort Lauderdale FL 33301 (US); Tomlin, Damian, Pembroke Pines FL 33025 (US)
(74) Representative: Belcher, Simon James

(56) References cited:
- US-A- 6 165 198
- US-A1- 2003 208 256
- US-B1- 6 468 266
- US-B1- 6 530 934

## Description

The present invention relates to a medical device for placing an embolic device at a predetermined site within a vessel of the human body, and more particularly, relates to a catheter-based deployment system for delivering an embolic device. This device is particularly suited to transport an embolic device, such as an embolic coil, through the tortious vasculature of the human brain to a selected site.

For many years, flexible catheters have been used to place various devices within the vessels of the human body. Such devices include dilation balloons, radiopaque fluids, liquid medications, and various types of occlusion devices such as balloons and embolic coils. Examples of such catheter-based devices are disclosed in US-5108407, entitled, "Method And Apparatus For Placement Of An Embolic Coil," and US-5122136, entitled, "Endovascular Electrolytically Detachable Guidewire Tip For The Electroformation Of Thrombus In Arteries, Veins, Aneurysms, Vascular Malformations And Arteriovenous Fistulas." These patents disclose catheter-based devices for delivering embolic coils to preselected positions within vessels of the human body in order to treat aneurysms, or alternatively, to occlude blood vessels at a particular location.

Coils which are placed in vessels may take the form of helically wound coils, or alternatively, may take the form of randomly wound coils, coils wound within coils or other such coil configurations. Examples of various coil configurations are disclosed in US-5334210, entitled "Vascular Occlusion Assembly;" and US-5382259, entitled, "Vasoocclusion Coil with Attached Tubular Woven or Braided Fibrous Covering." Examples of detachable helical coil are disclosed in US-6 468 266. Embolic coils are generally formed of a radiopaque metallic material, such as platinum, gold, tungsten, or alloys of these metals. Often, several coils are placed at a given location to occlude the flow of blood through the vessel, or aneurysm, by promoting thrombus formation at the particular site.

In the past, embolic coils have been placed within the distal end of a catheter. When the distal end of the catheter is properly positioned, the coil may then be pushed out of the end of the catheter with a pusher member to release the coil at the desired location. This procedure for placement of an embolic coil is conducted under fluoroscopic visualization such that the movement of the coil through the vasculature of the body may be monitored and the coil placed at the desired location.

Another procedure involves the use of glue or solder for attaching the coil to a guidewire, which in turn, is placed within a flexible catheter for positioning the coil within the vessel at a preselected position. Once the coil is in the desired position, the coil is held in position by the catheter and the guidewire is pulled proximally to thereby cause the coil to become detached from the guidewire and released from the catheter. Such a coil positioning system is disclosed in US-5263964 entitled, "Coaxial Traction Detachment Apparatus and Method."

Still another coil positioning procedure is that of having a catheter with a socket at the distal end of the catheter for retaining a ball which is, in turn, bonded to the proximal end of the coil. The ball, which is generally larger in diameter than the outside diameter of the coil, is placed in the socket within the lumen at the distal end of the catheter and the catheter is then moved into a vessel in order to place the coil at a desired position. Once the position is reached, a pusher wire with a piston at the end thereof is pushed distally from the proximal end of the catheter to push the ball out of the socket in order to release the coil at the desired position. Such a system is disclosed in US-5350397, entitled, "Axially Detachable Embolic Coil Assembly."

Another procedure for placing an embolic coil within a vessel is that of using a heat releasable adhesive bond for retaining the coil at the distal end of the catheter. One such system uses laser energy transmitted through a fiber optic cable to apply heat to the adhesive bond in order to release the coil from the end of the catheter. Such a procedure is disclosed in the aforementioned US-5108407.

Yet another coil deployment system incorporates a catheter having a lumen throughout the length of the catheter and a distal tip for retaining the coil for positioning the coil at a preselected site. The distal tip of the catheter is formed of a material which exhibits the characteristic that when the lumen of the catheter is pressurized the distal tip expands radially to release the coil at the preselected site. Such a deployment system is disclosed in US-6113622, entitled, "Embolic Coil Hydraulic Deployment System."

Still another coil deployment system incorporates an interlocking mechanism on the coil. The interlocking end on the embolic coil couples with a similar interlocking mechanism on a pusher assembly. A control wire which extends through the locking mechanism secures the coil to the pusher assembly. The pusher assembly and embolic coil are initially disposed within the lumen of a catheter. When the embolic coil is pushed out of the end of the catheter for placement, the control wire is retracted and the coil disengages from the pusher assembly. Such a deployment system is disclosed in US-5925059, entitled, "Detachable Embolic Coil Assembly."

Yet another coil deployment system incorporates an embolic device detachably mounted on the distal portion of a pusher member and held in place with a connector thread or fiber. The fiber passes through a cutter member that may be activated to cut the connector fiber. Once the connector fiber is cut, the embolic device is released. Such a deployment system is disclosed in US-A-2002/0165569, entitled, "Intravascular Device Deployment Mechanism Incorporating Mechanical Detachment."

Still another coil deployment system incorporates an embolic device with a stretch resistant member therethrough. The distal end of the stretch resistant member attaches to the embolic coil and the proximal end of the stretch resistant member is detachably mounted on the pusher member through various means such as adhesive, or by a connector fiber adhered to or tied to the pusher member, and is detachable by the application of heat. Such a deployment system is disclosed in US-A-2004/0034363, entitled, "Stretch Resistant Therapeutic Device."

Still another coil deployment system incorporates a pusher wire with a stiff wavy-shaped end segment which is coupled to the embolic coil and is placed in the lumen of the catheter. The coil is advanced through the catheter until it reaches a predetermined site in the vessel at which time the pusher wire is retracted and the embolic coil is released. Such a system is disclosed in US-6203547, entitled, "Vaso-occlusion Apparatus Having A Manipulable Mechanical Detachment Joint And A Method For Using The Apparatus."

Still another embolic device deployment system includes an elongated flexible pusher member slidably disposed within a lumen of a catheter. The embolic device is retained at the end of the pusher member with a detachment filament. When the embolic device is advanced to the predetermined site within the vessel, the detachment filament is withdrawn releasing the embolic device.

The present invention is directed toward a vasooclusive embolic device deployment system for use in placing an embolic device at a predetermined site within a vessel including an elongated flexible catheter and an elongated pusher member, preferably having a lumen therethrough and being slidably disposed within the catheter. An embolic device, preferably taking the form of a helically wound embolic coil having a plurality of turns, is releasably coupled to the distal end of the pusher member. The detachment system also includes a detachment filament which extends through the lumen of the catheter, or preferably through the lumen of the pusher member. The detachment filament extends around a turn of the embolic coil and then back through the lumen of the catheter, or preferably through a lumen of the pusher member. When the embolic coil is at the predetermined site within the vessel, the detachment filament may be pulled proximally to decouple the detachment filament from engagement of the turn of the embolic coil to thereby release the embolic coil.

In accordance with another aspect of the present invention, the vasooclusive embolic device deployment system includes a retaining clamp assembly mounted on the proximal end of the pusher member. The retaining clamp preferably takes the form of an adjustable chuck which applies a clamping pressure to the fiber to retain the filament until the chuck is opened to release the filament. The detachment filament extends through the clamp then through and the lumen of the pusher member and then around a turn of the embolic coil, and then back through the lumen of the pusher member and through the retaining clamp. Upon loosening the retaining clamp, one of the ends of the detachment filament may be pulled proximally to decouple the detachment filament from the embolic coil to thereby release the embolic coil at the predetermined site.

Embodiments of the invention will now be described by way of example only with reference to the accompanying drawings, in which:
Figure 1 is an enlarged, partially sectional view of an embodiment of an embolic device deployment system in accordance with the present invention;
Figure 2 is an enlarged, sectional view, illustrating in more detail the coil deployment system of Figure 1; and
Figures 3, 3a, 3b, and 3c are enlarged, sectional views of the coil deployment system shown in Figures 1 and 2 illustrating the sequential steps in the advancement of the embolic device, removal of a detachment filament, and release of the embolic device.

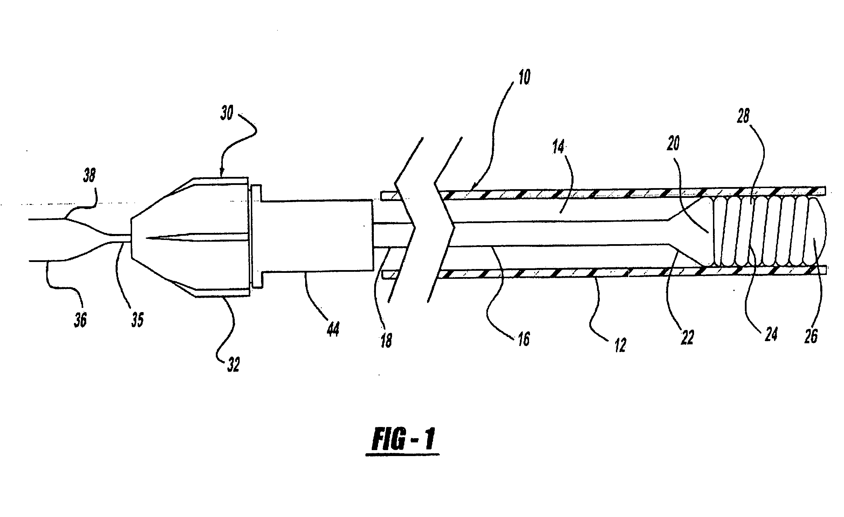

Figure 1 generally illustrates one embodiment of an embolic device deployment system 10 of the present invention having an elongated flexible catheter 12 having a lumen 14 extending therethrough. An elongated flexible pusher member 16 is slidably disposed within the lumen 14 of the catheter 12. The pusher member 16 includes a proximal end 18 and a distal end 20, and the distal end 20 includes a tip portion 22 having an increase in diameter. Retractably mounted on the pusher member 16 at its distal end 20 is an embolic device, which preferably takes the form of an embolic coil 24, formed of a plurality of helical turns 28 connected to an atraumatic distal bead 26. While the embolic coil 24 is a preferred configuration of the embolic device, alternative device configurations are suitable such as embolic filaments, braids, expandable meshes, foams and stents. The tip portion 22 at the distal end 20 of the pusher member 16 engages the embolic coil 24. Mounted on the proximal end 18 of the pusher member 16 is a retaining clamp assembly 30, which includes a cap 32 which engages a chuck 44. A detachment filament 35 having ends 36 and 38 extends through the retaining clamp assembly 30 and serves to retain the embolic coil 24 in position at the distal end 20 of the elongated pusher member 16.

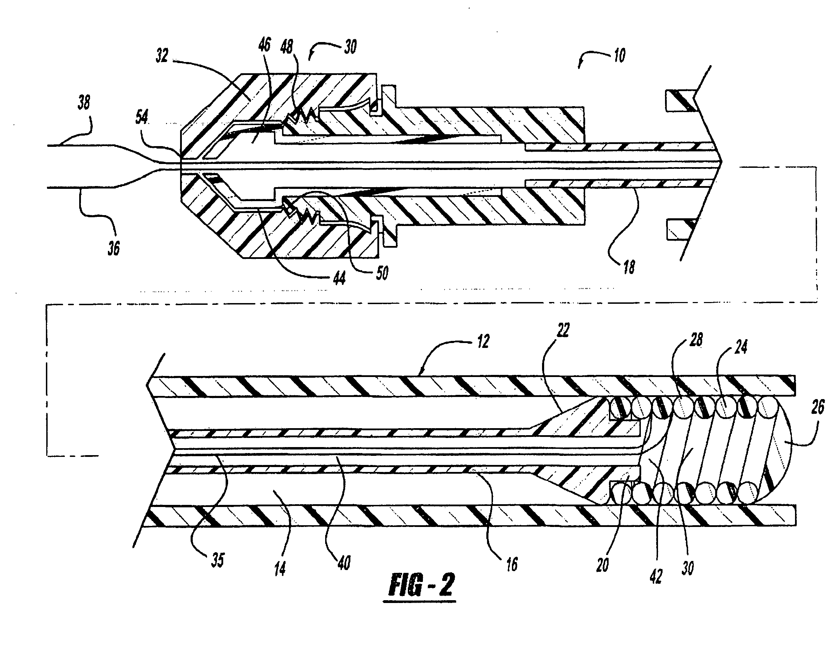

Figure 2 illustrates in more detail the configuration of the embolic device deployment system 10 of Figure 1. The pusher member 16, preferably has a lumen 40 therethrough and is slidably disposed within the lumen 14 of the catheter 12. Preferably, the pusher member 16 is constructed from a nickel titanium based shape memory alloy, such as nitinol, but alternatively, the pusher member may be constructed from many materials that are pushable and flexible such as stainless steel, nylon, PTFE, other metals or polymers and composites. Additionally, the pusher member 16 should have an outside diameter in the range of about 0.002 to 0.020 centimeters.

As shown, the embolic coil 24 is helically wound but may take various other forms. Such as for example, a randomly wound coil. The distal bead 26 is connected to the distal end of the embolic coil 24. The embolic coil 24 also includes a lumen 42 extending therethrough created by the plurality of helical turns 28. The diameter of the lumen 42 of the embolic coil 24 is slightly greater than the diameter of the distal end 20 of the pusher member 16. With this configuration, the embolic coil 24 may be pushed distally by the distal end 20 of the pusher member 16.

A detachment filament 35 includes ends 36 and 38 which extend proximally from the proximal end of the clamp assembly 30. The detachment filament 35 extends through the retaining clamp assembly 30 and through the lumen 40 of the pusher member 16. The detachment filament 35 also loops around one of the plurality of helical turns 28 of the embolic coil 24 and is returned through the lumen 40 of the pusher member 16, exits the pusher member 16 and extends through the lumen 46 of the chuck 44 of the clamp assembly 30 and then exits at the proximal end of the clamp assembly 30. The cap 32 applies pressure to the chuck 44, such that the chuck 44 applies squeezing pressure to the ends 36 and 38 of the detachment filament 35 thereby preventing movement of the detachment filament 35. The embolic coil 24 may not be disengaged from the distal end 20 of the pusher member 16, so long as the cap 32 remains tight on the chuck 44 and the detachment filament 35 is secured relative to the pusher member 16. Finally, the detachment filament 35 is preferably constructed of nitinol, but alternatively may be formed from various other materials such as platinum, nylon, PTFE, flexible metals, polymers, or composites. Preferably, the material used for the detachment filament 35should be very flexible, have a high tensile strength and a low elongation when a tensile force is applied to the filament. The diameter of the detachment filament 35 is in the range of about 0.001 to 0.090 centimeters and preferably on the order of about 0.002 to 0.020 centimeters.

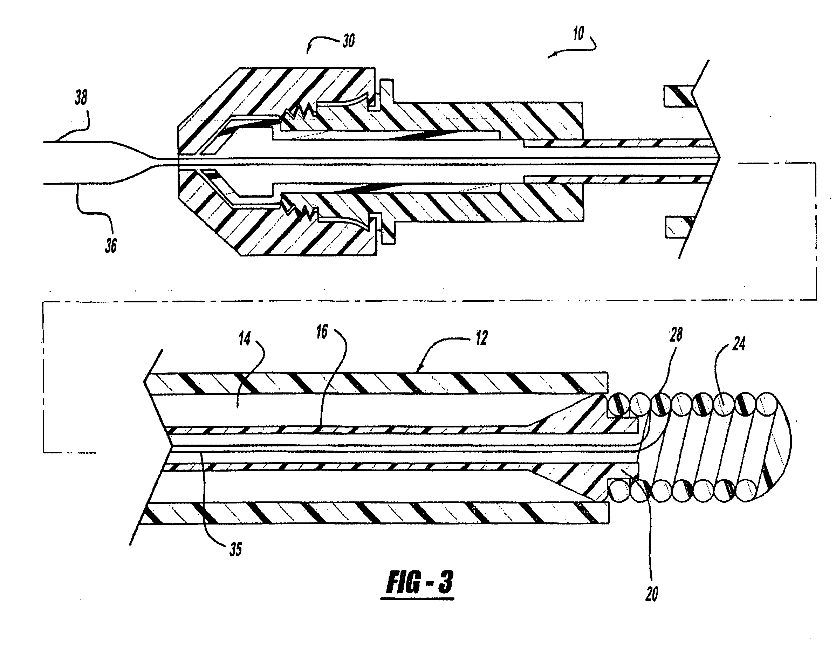

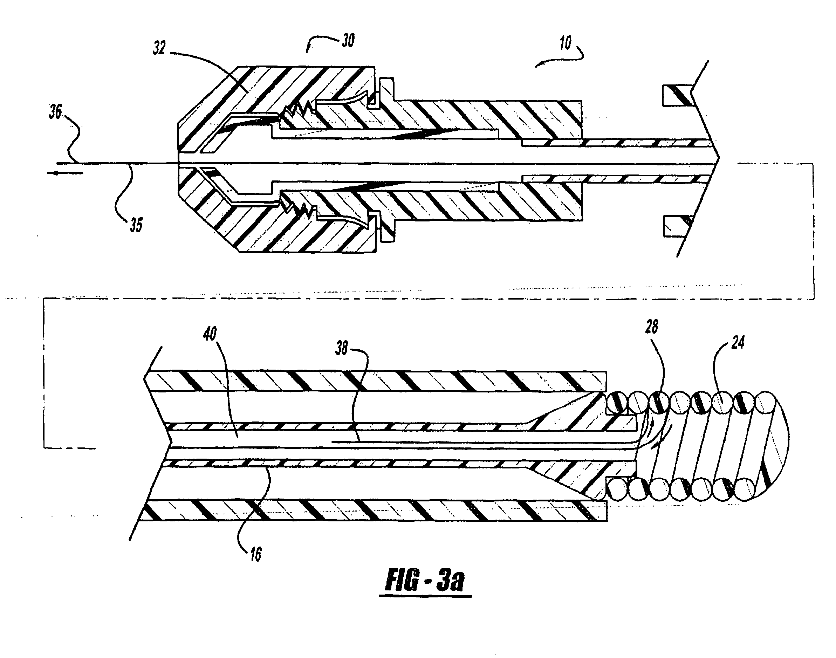

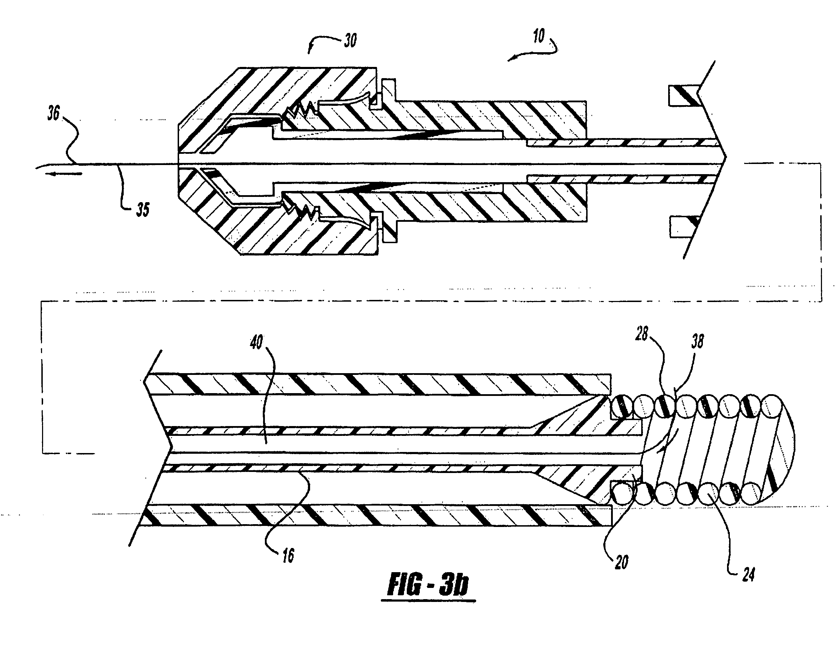

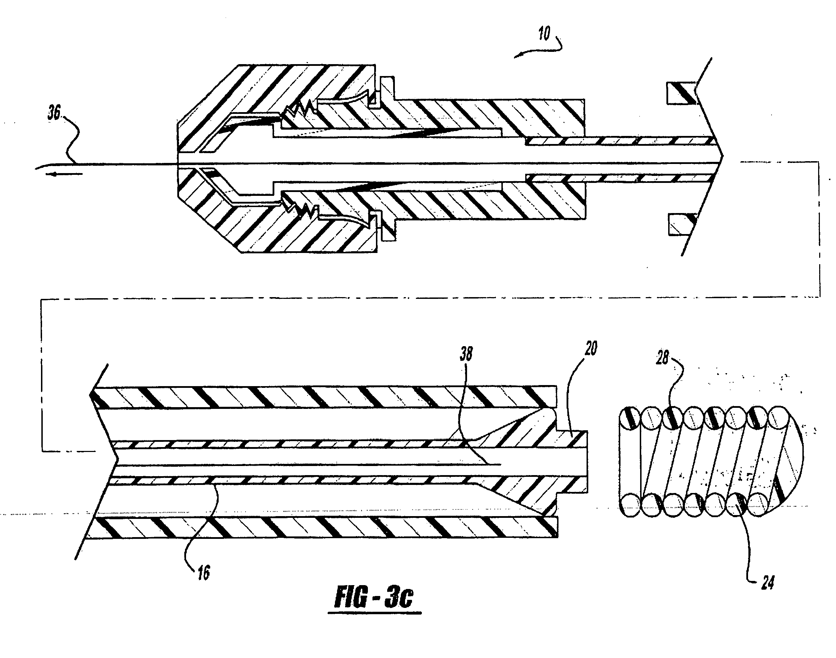

Figures 3, 3a, 3b, and 3c generally illustrate the operation of the embolic device deployment system 10 and demonstrate the detachment filament release mechanism. More particularly, Figure 3 illustrates the catheter 12 positioned at a predetermined location, analogous to placement in a vessel and the pusher member 16 advanced through the lumen 14 of the catheter 12, such that the embolic coil 24 exits the distal end of the catheter 12. In addition, the retaining clamp assembly 30 maintains tension on the ends 36 and 38 of detachment filament 35, such that the embolic coil 24 is retained at the distal end 20 of the pusher member 16.

Figure 3a illustrates the embolic device deployment system 10 with the embolic coil 24 positioned at a desired location adjacent the distal section of the catheter 12. The cap 32 is loosened to permit one end 36 of the detachment filament 35 to be pulled proximally. As the end 36 of the detachment filament 35 is pulled proximally from the retaining clamp assembly 30, the other end 38 of the detachment filament 35 moves distally through the lumen 40 of the pusher member 16.

Figure 3b illustrates the embolic device deployment system 10 with the end 36 of the detachment filament 35 pulled further proximally from the retaining clamp assembly 30 and the other end 38 of the detachment filament 35 withdrawn from its position around one of the plurality of helical turns 28 of the embolic coil 24.

Figure 3c illustrates the embolic device deployment system 10 with the end 38 of the detachment filament 35 completely removed from the helical turn 28 of the embolic coil 24. Finally, the embolic coil 24 disengages from the distal end 20 of the pusher member 16 and is released at the predetermined site within the vessel.

As is apparent, there are numerous modifications of the preferred embodiment described above which will be readily apparent to one skilled in the art, such as many variations and modifications of the coil including numerous coil winding configurations, or alternatively other types of implant devices. There are obviously variations in the path and attachment of the detachment filament. Additionally, the retaining clamp assembly could also be modified with other methods used to apply pressure to the detachment filament ends. These modifications would be apparent to those having ordinary skill in the art to which this invention relates and are intended to be within the scope of the claims which follow.

## Claims

1. A vasooclusive embolic device deployment system (10) for use in placing an embolic device at a predetermined site within a vessel comprising:
an elongated flexible catheter (12) having a lumen (14) extending therethrough and having proximal (18) and distal ends (20);
an elongated pusher member (16) having proximal (18) and distal (20) ends and being slidably disposed within the lumen of the catheter;
an embolic device (24) having a plurality of turns releasably engaging the distal end of the pusher member; and,
a detachment filament (35) extending from a position proximal (36) of the proximal end of the catheter through the lumen of the catheter and around a turn of the embolic device, such that when the embolic device is properly positioned at a predetermined site within the vessel the detachment filament may be pulled proximally to decouple the detachment filament from the turn of the embolic device to thereby release the embolic device (24) at the predetermined site.

2. A vasooclusive embolic device (10) deployment system as defined in Claim 1, wherein said pusher member includes a lumen (40) extending therethrough, and said detachment filament (35) extends from a position proximal of the of the proximal end of the pusher member (16) through said lumen of the pusher and around a turn of the embolic device, such that when the embolic device is properly positioned at a predetermined site within the vessel the detachment filament may be pulled proximally to decouple the detachment filament from the turn of the embolic device (24)

3. A vasooclusive embolic device deployment system (10) as defined in Claim 1, including a retaining clamp (30) having a lumen extending therethrough and having distal and proximal ends, and bein mounted on the proximal end of the pusher member (16), and wherein said detachment filament (35) extends from a position proximal of the proximal end of the clamp (30) through the lumen of the clamp and through the lumen of the catheter and around a turn of the embolic device (24).

4. A vasooclusive embolic device deployment system (10) as defined in Claim 3, wherein the retaining clamp comprises a chuck (44) having a tightening cap (32) for controlling pressure applied to the detachment filament (35).

5. A vasooclusive embolic device deployment system (10) as defined in Claim 2, including a retaining clamp (30) having a lumen (40) extending therethrough and having distal and proximal ends, and being mounted on the proximal end of the pusher member (16), and wherein said detachment filament (35) extends from a position proximal of the proximal end of the clamp through the lumen of the clamp and through the lumen of the pusher member and around a turn of the embolic device (24).

6. A vasooclusive embolic device deployment system (10) as defined in Claim 5, wherein the retaining clamp comprises a chuck (44) having a tightening cap (32) for controlling pressure applied to the detachment filament (35).

7. A vasooclusive embolic device deployment system (10) as defined in Claim 1, wherein the detachment filament is formed of nitinol.

8. A vasooclusive embolic device deployment system (10) as defined in Claim 1, wherein the embolic device is helically wound.

9. A vasooclusive embolic device deployment system (10) as defined in Claim 2, wherein the detachment filament is formed of nitinol.

10. A vasooclusive embolic device deployment system (10) as defined in Claim 2, wherein the embolic device is helically wound.

## Patentansprüche

1. Vasookklusives Entfaltungssystem für eine Embolievorrichtung (10) zur Verwendung beim Plazieren einer Embolievorrichtung an einem vorbestimmten Ort innerhalb eines Gefäßes, das folgendes aufweist:
einen länglichen flexiblen Katheter (12) mit einem Lumen (40), das sich durch diesen erstreckt, und mit einem proximalen (18) und einem distalen (20) Ende;
ein längliches Druckelement (16) mit einem proximalen (18) und einem distalen (20) Ende, das gleitfähig in dem Lumen des Katheters angeordnet ist;
eine Embolievorrichtung (24) mit mehreren Windungen, die lösbar an dem distalen Ende des Druckelements angreifen; und
einen Freisetzungsfaden (35), der sich von einer Position proximal (36) des proximalen Endes des Katheters durch das Lumen des Katheters und um eine Windung der Embolievorrichtung erstreckt, so daß, wenn die Embolievorrichtung an einem vorbestimmten Ort in dem Gefäß richtig positioniert ist, der Freisetzungsfaden proximal gezogen werden kann, um den Freisetzungsfaden von der Windung der Embolievorrichtung zu trennen, um dabei die Embolievorrichtung (24) an dem vorbestimmten Ort zu lösen.

2. Vasookklusives Entfaltungssystem für eine Embolievorrichtung (10) nach Anspruch 1, wobei das Druckelement ein Lumen (40) aufweist, das sich durch dieses erstreckt, und der Freisetzungsfaden (35) sich von einer Position proximal des proximalen Endes des Druckelements (16) durch das Lumen des Drückers und um eine Windung der Embolievorrichtung erstreckt, so daß, wenn die Embolievorrichtung an dem vorbestimmten Ort in dem Gefäß richtig positioniert ist, der Freisetzungsfaden proximal gezogen werden kann, um den Freisetzungsfaden von der Windung der Embolievorrichtung (24) zu trennen.

3. Vasookklusives Entfaltungssystem für eine Embolievorrichtung (10) nach Anspruch 1, das eine Halteklammer (30) umfaßt, die ein Lumen, das sich durch diese erstreckt, und ein distales und ein proximales Ende aufweist, und auf dem proximalen Ende des Druckelements (16) montiert ist, und wobei der Freisetzungsfaden (35) sich aus einer Position proximal des proximalen Endes der Klammer (30) durch das Lumen der Klammer und durch das Lumen des Katheters und um eine Windung der Embolievorrichtung (24) erstreckt.

4. Vasookklusives Entfaltungssystem für eine Embolievorrichtung (10) nach Anspruch 3, wobei die Halteklammer ein Futter mit einer Festspannkappe (32) zum Steuern des Drucks, der auf den Freisetzungsfaden (35) ausgeübt wird, aufweist.

5. Vasookklusives Entfaltungssystem für eine Embolievorrichtung (10) nach Anspruch 2, das eine Halteklammer (30) umfaßt, die ein Lumen (40), das sich durch diese erstreckt und ein distales und ein proximales Ende aufweist, und die auf dem proximalen Ende des Druckelements (16) montiert ist, und wobei der Freisetzungsfaden (35) sich von einer Position proximal des proximalen Endes der Klammer durch das Lumen der Klammer und durch das Lumen des Druckelements und um eine Windung der Embolievorrichtung (24) erstreckt.

6. Vasookklusives Entfaltungssystem für eine Embolievorrichtung (10) nach Anspruch 5, wobei die Halteklammer ein Futter (44) mit einer Festspannkappe (38) zum Steuern des Drucks, der auf den Freisetzungsfaden (35) ausgeübt wird, umfaßt.

7. Vasookklusives Entfaltungssystem für eine Embolievorrichtung (10) nach Anspruch 1, wobei der Freisetzungsfaden aus Nitinol gebildet ist.

8. Vasookklusives Entfaltungssystem für eine Embolievorrichtung (10) nach Anspruch 1, wobei die Embolievorrichtung spiralförmig gewunden ist.

9. Vasookklusives Entfaltungssystem für eine Embolievorrichtung (10) nach Anspruch 2, wobei der Freisetzungsfaden aus Nitinol gebildet ist.

10. Vasookklusives Entfaltungssystem für eine Embolievorrichtung (10) nach Anspruch 2, wobei die Embolievorrichtung spiralförmig gewunden ist.

## Revendications

1. Système de déploiement (10) d'un dispositif embolique vaso-occlusif destiné à être utilisé pour placer un dispositif embolique sur un site prédéterminé dans un vaisseau, comprenant :
■ un cathéter flexible allongé (12) ayant une lumière (14) s'étendant à travers celui-ci et ayant des extrémités proximale (18) et distale (20) ;
■ un élément poussoir allongé (16) ayant des extrémités proximale (18) et distale (20) et étant disposé de manière coulissante dans la lumière du cathéter ;
■ un dispositif embolique (24) ayant une pluralité de spires mettant en prise de manière amovible l'extrémité distale de l'élément poussoir ; et
■ un filament de détachement (35) s'étendant à partir d'une position proximale (36) de l'extrémité proximale du cathéter par la lumière du cathéter et autour d'une spire du dispositif embolique, de sorte que lorsque le dispositif embolique est correctement positionné sur le site prédéterminé dans le vaisseau, le filament de détachement peut être tiré de manière proximale pour découpler le filament de détachement de la spire du dispositif embolique pour libérer ainsi le dispositif embolique (24) sur le site prédéterminé.

2. Système de déploiement (10) d'un dispositif embolique vaso-occlusif selon la revendication 1, dans lequel ledit élément poussoir comprend une lumière (40) s'étendant à travers celui-ci, et ledit filament de détachement (35) s'étend à partir d'une position proximale de l'extrémité proximale de l'élément poussoir (16) par ladite lumière du poussoir et autour d'une spire du dispositif embolique, de sorte que lorsque le dispositif embolique est correctement positionné sur un site prédéterminé dans le vaisseau, le filament de détachement peut être tiré de manière proximale pour découpler le filament de détachement de la spire du dispositif embolique (24).

3. Système de déploiement (10) d'un dispositif embolique vaso-occlusif selon la revendication 1, comprenant un dispositif de serrage de retenue (30) ayant une lumière s'étendant à travers celui-ci et ayant des extrémités distale et proximale, et étant monté sur l'extrémité proximale de l'élément poussoir (16) et dans lequel ledit filament de détachement (35) s'étend à partir d'une position proximale de l'extrémité proximale du dispositif de serrage (30) à travers la lumière du dispositif de serrage et à travers la lumière du cathéter et autour d'une spire du dispositif embolique (24).

4. Système de déploiement (10) d'un dispositif embolique vaso-occlusif selon la revendication 3, dans lequel le dispositif de serrage de retenue comprend un mandrin (44) ayant un capuchon de serrage (32) pour contrôler la pression appliquée sur le filament de détachement (35).

5. Système de déploiement (10) d'un dispositif embolique vaso-occlusif selon la revendication 2, comprenant un dispositif de serrage de retenue (30) ayant une lumière (40) s'étendant à travers celui-ci et ayant des extrémités distale et proximale, et étant monté sur l'extrémité proximale de l'élément poussoir (16), et dans lequel ledit filament de détachement (35) s'étend à partir d'une position proximale de l'extrémité proximale du dispositif de serrage à travers la lumière du dispositif de serrage et à travers la lumière de l'élément poussoir et autour d'une spire du dispositif embolique (24).

6. Système de déploiement (10) d'un dispositif embolique vaso-occlusif selon la revendication 5, dans lequel le dispositif de serrage de retenue comprend un mandrin (44) ayant un capuchon de serrage (32) pour contrôler la pression appliquée sur le filament de détachement (35).

7. Système de déploiement (10) d'un dispositif embolique vaso-occlusif selon la revendication 1, dans lequel le filament de détachement est formé à partir de Nitinol.

8. Système de déploiement (10) d'un dispositif embolique vaso-occlusif selon la revendication 1, dans lequel le dispositif embolique est enroulé de manière hélicoïdale.

9. Système de déploiement (10) d'un dispositif embolique vaso-occlusif selon la revendication 2, dans lequel le filament de détachement est formé à partir de nitinol.

10. Système de déploiement (10) d'un dispositif embolique vaso-occlusif selon la revendication 2, dans lequel le dispositif embolique est enroulé de manière hélicoïdale.
